(19) Europäisches Patentamt / European Patent Office / Office européen des brevets

(11) **EP 4 419 013 B1**

(12) **EUROPEAN PATENT SPECIFICATION**

(45) Date of publication and mention
of the grant of the patent:
**07.01.2026 Bulletin 2026/02**

(21) Application number: **21810717.5**

(22) Date of filing: **18.10.2021**

(51) International Patent Classification (IPC):
*A61B 10/00* (2006.01)　　*A61F 13/38* (2006.01)
*A61B 10/02* (2006.01)

(52) Cooperative Patent Classification (CPC):
**A61B 10/0045; A61F 13/38;** A61B 2010/0074;
A61B 2010/0216

(86) International application number:
**PCT/IB2021/059576**

(87) International publication number:
**WO 2023/067368 (27.04.2023 Gazette 2023/17)**

(54) **SAMPLING FLEXIBLE CLOTH WITH RUPTURE ZONE, WHICH PROTECTS THE SAMPLED CELLS**

FLEXIBLES ENTNAHMEGEWEBE MIT BRUCHZONE ZUM SCHUTZ DER ENTNOMMENEN ZELLEN

TISSU SOUPLE D'ÉCHANTILLONNAGE AVEC ZONE DE RUPTURE, QUI PROTÈGE LES CELLULES ÉCHANTILLONNÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**TN**

(43) Date of publication of application:
**28.08.2024 Bulletin 2024/35**

(73) Proprietor: **Chaffringeon, Bernard-Marie**
**110354 Pitesti (RO)**

(72) Inventors:
• **CHAFFRINGEON, Bernard-Marie**
**Dubai (AE)**

(74) Representative: **Preda, Raluca-Laura**
**Rominvent S.A.**
**35, Ermil Pangratti Str.**
**1st Floor**
**Sector 1**
**011882 Bucuresti (RO)**

(56) References cited:
WO-A1-99/21521　　　　DE-U1- 202009 016 553
US-A- 5 121 752　　　　US-A1- 2010 286 552
US-A1- 2018 161 019　　US-A1- 2018 353 737
US-A1- 2020 390 425

## Description

The present invention relates to the field of sampling flexible cloths and swab applicators used with said sampling cloth for sampling specimens and/or culture material such as cells, cell residues, proteins, DNA, RNA, and/or other material from human or animal bodily cavities such as the vaginal cavity, rectal cavity, oral or nasal cavity. More specifically, the present invention relates to a swab kit comprising a sampling, flexible cloth with rupture zone for sampling a specimen from a human or animal bodily cavity by means of a swab applicator and a swab applicator comprising a bent section to be used with said cloth.

## Background of the invention

The swab sampling is the most commonly used and convenient sampling method during collection and transmittal of biological samples. Swab applicators are currently used with a sampling material such as cotton wool or fibrous material for sampling for specimens and/or culture material from human or animal bodily cavities such as the vaginal cavity, rectal cavity, oral or nasal cavity.

Conventional swab applicators include a longitudinal swab rod made of wood or a plastic material, a handle and a swab or sampling head at an end of the swab rod, opposite the handle. The sampling material is wrapped on the swab head. Such applicators may be kept in a sterile package until they are to be used. These swab applicators have dimensions suitable to allow an easy insertion inside a specific bodily cavity of a human or animal. For this reason, the swab applicator has to be long enough to ensure a secure hold with the user's fingers during insertion/removal and at the same time to allow the grip area to remain outside of the bodily cavity during the sampling process.

Cytology is defined as morphological analysis of cells for screening or diagnostic purposes. Cytology is a fundamental step in the cervical cancer screening process, as it enables detection of precancerous lesions, a pre-requisite to attaining a recovery rate of the order of 100% (before progression to invasive cancer). The accuracy of the cytological examination from any body site depends greatly on the quality of collection, preparation, staining and interpretation of the material. Inadequacy in any of these steps will adversely affect the quality of diagnostic cytology. Diagnostic accuracy and reliability are major issues in cytology practice. Over the years many quality control measures have been introduced for ensuring high standards in cytology. Among them the most important are regular continuing education of medical and technical personnel, certification and accreditation of laboratory, introduction of quality assurance and quality control measures, computerization, introduction of internationally accepted terminology, improvement of sample preparation techniques, quantitative and analytical cytology techniques and advanced technologies including automation.

Screening tests, particularly for cervical cancer, account for 80% of all cytology evaluations and are referred to as *gynaecological specimen testing.* The main focus of cyto-screening is cervical cancer. A common application of cytopathology is the Pap smear, a screening tool used to detect precancerous cervical lesions that may lead to cervical cancer. Collecting specimens such as cytological, DNA, RNA, viruses such as the Coronavirus or HPV, and/or biologic samples for testing, study and diagnosis, especially for cervical cancer or STIs usually requires a complex examination and an often painful sampling made by a trained specialist (physician or nurse). Such samplings for testing for cervical cancers or STIs are traditionally performed on women by a gynecologist by inserting a speculum into the patient's vagina in a manner to expose the cervix of the uterus and then inserting a cervical scraper (with a swab or a brush) for sampling tissue from the endocervical canal and cervical os. The sampling devices that are commonly used are: broom-type sampling device or an endocervical brush/plastic spatula device. Normal cells are cohesive in nature but exfoliated when they attain maturation. During malignant conditions or during infection, the exfoliation becomes exaggerated and the epithelial cells show variation in morphology. Such exfoliated cells, when collected and appropriately stained, give information on the living epithelium from which they are derived. These characteristic cellular and nuclear appearances in cells thrown off from healthy epithelium differ distinctly from those, derived from inflamed or malignant lesions. Thus by studying the alterations in morphology of the exfoliated cells and their pattern, the diagnosis of various pathologic conditions can be made.

The cervical scrapers are designed to scratch the tissue in order to take samples of tissue. Throughout this process, the woman must remain in a reclining (gynecological) position. The cervical scrapers are inserted into the vaginal tube to reach the endocervical canal or ectocervix and then the sampling device is rotated inside the bodily cavity in one direction to collect the specimen. The rotation must be done slowly and in a clockwise direction to reduce unnecessary bleeding. Finally, the obtained specimen is applied directly on a glass slide (process used by the conventional Pap smear), or placed into a recipient containing a liquid preservative (process referred to as liquid-based cytology (LBC)) in order to go through the analysis and evaluation steps. However, in about 30% of cases, unsatisfactory specimens are collected by this technique, leading to the need to repeat the procedure or to false or incomplete results. The experience of the person who is taking the smear is very important in getting smears with adequate cellular composition. The cervix must be clearly visualized and the entire transformation zone is scraped. The collection device may play an important role in sample adequacy. The shape, surface, texture and material of the device may determine how much of the scraped material is deposited and is available for screening and analysis.

Several methods of obtaining cytologic material from the uterine cervix are available. However, use of cotton swab for collection of cervical smear is to be discouraged, in view of the drying artifacts and loss of cells, which are caused by this method.

The current practice for proper positioning of the swab applicator inside a bodily cavity depends on the patient's anatomy and physician's skill. For example, when sampling for specimens and/or culture material for detection of cervical cancer, the insertion of the swab applicator into the vaginal cavity through the vaginal opening to reach into the cervix of the uterus with the swab for sampling tissue from the endocervical canal and cervical os is a challenging procedure to perform. This procedure is often done blindly, by advancing the applicator until the physician senses slight resistance to indicate that the tip of the applicator has approximated the cervical os (or *"ostium of uterus"* which means *orifice of the uterus*).

The vaginal cavity is a tube that tilts posteriorly between the urethra and rectum, with the urethra bound to its anterior wall. If standing, the vaginal tube will point in an upward-backward direction to form a 45-degree angle with the uterus and an about 60-degree angle to the horizontal. However, the exact angles are variable depending on individual anatomy and with contents of the bladder and colon.

The insertion of the longitudinal swab applicator into the vaginal cavity is done only with the use of a speculum which is placed into the patient's vagina in a manner to expose the cervix of the uterus. Even so, due to the reclining (gynecological) position of the women, the swab applicator must be inclined at an angle according to the particular position of the women during examination and of the individual anatomy. This is also done blindly, by inclining the applicator until the physician can reach the endocervical canal and cervical os to collect specimens and/or culture material.

This procedure can be uncomfortable for the women and/or the swab applicator can be difficult to insert into the appropriate region of the bodily cavity due to their rigidity and incapability of accommodating variations in anatomy, e.g., variations in the size, shape and orientation of the bodily cavity among patients, or postoperative distortions in anatomy. Also, improper swab applicator placement may increase the risk of tissue perforation or damage during insertion into the bodily cavity and unsatisfactory sampling for specimens and/or culture material.

Another potential disadvantage with this known swab applicator for taking a culture sample test is that it is possible for the doctor or nurse who takes the test to contaminate the test sample by the handling of the swab applicator. This may also bring in bacteria and thus affecting the test results.

Recent developments in this field have resulted in a breakable swab applicator which can easily break or separate the swab head from the swab rod after the sampling of a specimen, without using scissors. CN204106055U, CN205215271U, CN206818436U utility model applications disclose a breakable swab applicator which comprises a swab rod, a handle and a sampling head. The swab rod is provided with at least an easy-breaking concave groove. After the sampling is done, the swab applicator's head is placed into the open end of a vial and the testing personnel only need to press the swab rod to break the swab head from the swab rod and seal the vial to be sent to the testing laboratory.

Document WO1999021521A1 describes a device and methods for obtaining samples of cellular material from the vaginal introitus which, typically, vaginal fluid is adsorbed by a body through a semi-permeable membrane which retains the cellular material. In one form, the device comprises a tampon-like body having a highly adsorbent portion and a soft but less permeable proximal portion. A semi-permeable membrane is fitted over the body. A string is provided to withdraw the body from the vaginal introitus after it has been indwelling therein for some minutes or hours. Fluid imbibed by the body must pass through the membrane and leave behind any cellular material. Upon withdrawal of the device, the filter-like membrane is inverted to protect the sample material from contact with the wall of the vaginal canal. The membrane can be easily removed from the device and the sample material recovered for analysis.

Document US20200390425A1 describes a self-sampling kit and method for the universal sampling of at least one specimen such as a cell, cell residue, DMA, RNA, protein, virus, bacterium, parasite or fungus from the vaginal and/or rectal cavities of humans and animals, said kit comprising a self-sampling cloth made of a flexible fabric with an absorbancy of 3.5 g/g or less and a sealable recipient for storing and transporting said sampling cloth.

Document US20180161019A1 describes various embodiments of a swab device, system, and method provided for measuring and assessing saliva flow in the mouth of a person. In one embodiment, a system includes a swab device and a measuring device. The swab device includes a handle portion and a swab portion, the handle portion being elongated to extend between a proximal end and a distal end. The swab portion is coupled to a distal portion of the handle portion and is sized and configured to collect saliva from the mouth. The measuring device is sized and configured to measure an amount of the saliva collected with the swab portion, the measuring device including a nesting portion integrated therewith. Such nesting portion of the measuring device is sized and configured to receive the proximal end of the handle portion of the swab device to measure the saliva collected therewith.

Document US20180353737A1 describes a morpho-anatomic flexible web intended to be introduced into a vaginal or rectal cavity of a human or animal, being provided with means of removal from said cavity, the web having a proximal end which corresponds to the part of the web that first contacts the cavity and a distal end which corresponds to the part of the web that last enters the cavity. The web comprises two sheets that at least partly overlap, defining an overlapping area, wherein said first and second sheets are fixed to one another by means of at least two stopping elements, defining at least one pouch in the interior of the overlapping area for holding a substance, and wherein the web further comprises at least one blocking element for retaining an applicator in a substantially fixed position relative to the web, the blocking element having at least one blocking side which is perpendicular to the direction of insertion of the web into the vaginal or rectal cavity, preferably the blocking side being situated below the mouth of the pouch such that part of the web may fold over the mouth of the pouch to completely close it.

Another document DE202009016553U1 describes a test device for manually examining the properties of a body fluid with an assayable overstressing agent, test strip having an indicating agent, in particular for measuring the pH of the vaginal fluid and a double-sided adhesive tape for securing the test strip to a portion of the protective means and for protecting the test strip prior to use. The test device consists of three parts: a single envelope of rectangular cut with at least one glued test strip, an insertion pin which is inserted in the single envelope and an outer protective sleeve, which receives the single cover with the insertion pin and serves as a handle.

Another document US5121752A describes an apparatus and method of obtaining self-administered PAP smears which includes a hollow, cylindrical speculum of suitable minimum inside diameter to permit mirror vision therethrough. The speculum is formed with an angled front orifice wherein the front angle between the orifice and the axis of the speculum is particularly designed to easily fit about the cervix. The entire squamocolumnar junction of the uterine cervix is exposed within the front orifice to permit complete wiping, preferably under visual observation through a hand mirror. A hinged, elongated spatula and an angled endocervical sampler are included as part of the test equipment to faciliate the taking of uncontaminated, accurate specimens.

Yet another document US2010286552A1 describes a wiping device for wiping the surface of a cavity, comprising an implement having a distal end dimensioned and configured with a rounded tip to facilitate insertion of the device into the cavity, the implement also having a proximal end configured for manually grasping the implement and for inserting the distal end of the implement into the cavity, and a flexible wiping cloth configured to be received over the distal end of the implement, to conform to its rounded tip, and to define a lower edge to be releasable engaged by said implement in order to be releasably retained on the implement.

However, a disadvantage of the known swab applicators with a break groove opened to the outside of the swab rod is that it is easy to cause damage to the tissue of the sampled organ when inserting or removing the swab applicator into/out of the bodily cavity due to the sharp edges of the grooves.

Also, the known swab applicators with easy-breaking grooves have only one breaking point/surface. Therefore, a certain degree of stress is put on the trained specialist (physician or nurse) while using the known swab applicators. If the insertion force is increased above a certain level, the accidental breaking of the swab head inside the bodily cavity during the sampling process can occur, if not handled with caution.

Still, the known breakable swab applicators do not solve the above mentioned problem which relates to the insertion of the longitudinal swab applicator into the bodily cavity according to the particular position of the patient during sampling/examination and of the individual anatomy without causing discomfort and/or increase the risk of tissue perforation or damage during insertion into the bodily cavity and unsatisfactory sampling for specimens and/or culture material.

**Technical problem**

Therefore, the object of the present invention is to eliminate the disadvantages presented above by providing an inexpensive swab applicator comprising a bent section which can be used with any sampling material, or a swab kit comprising an applicator and a sampling cloth, with improved precision rate and efficacy in sampling, and preserving specimens like cells, cell residues, DNA, RNA, proteins, viruses, bacteria, parasites, or fungi and/or other materials of interests from the bodily cavities of humans or animals taking into account the particular position of the patient during sampling/examination and of the individual anatomy without causing discomfort and without damaging the tissue of the sampled organ or accidentaly break the swab head of the swab applicator during insertion into the bodily cavity.

This aim is achieved by a swab kit comprising a swab applicator comprising a bent section which can be used with a sampling cloth according to the present invention as described below.

**Definitions**

By *sampling* it is meant a process of harvesting specimens wherein the sampling procedure can be performed, for example, by a professional, such as a doctor or a nurse, by an individual on him - or on herself, or by an individual on another individual without the assistance from a trained professional.

By *specimen* it is meant any cell, cell residue, DNA, RNA, protein, virus, bacterium, parasite, fungus and/or other material of interests.

A *direction of insertion (x)* of the swab applicator is defined, which is a longitudinal axis of the swab applicator and being oriented from the end of the swab applicator that remains outside the bodily cavity, called *distal end of the swab applicator,* to the end of the swab applicator that first contacts said cavity, called *proximal end of the swab applicator.* When looking in the direction of insertion, the *top* of the swab applicator or the *upper part* is represented by the extremity of the swab applicator that comprises its proximal point; that is the point that first contacts the bodily cavity. At the same time, the *bottom* of the swab applicator or the *lower part* is represented by the extremity of the swab applicator that comprises its distal end; that is the point of the swab applicator opposite to the proximal point, i.e. placed at the furthest distance from the proximal point on the insertion direction. Taking into account the above definitions, then *lateral sides* of the swab applicator will be the left and right extremities, considering the top and bottom as defined above.

The sampling cloth according to the invention is designed to be inserted into the bodily cavity of a human or animal, by means of the swab applicator. Thus, a *direction of insertion of the sampling cloth* is defined, which is a longitudinal axis of the sampling cloth and being oriented from the end of the sampling cloth that last enters the bodily cavity or remains outside the cavity, called *distal end of the sampling cloth,* to the end of the sampling cloth that first enters said bodily cavity, called *proximal end of the sampling cloth.* When looking in the direction of insertion, the *top of the sampling cloth* or *the upper part* is represented by the extremity of the sampling cloth that comprises its *proximal point;* that is the point that first enters the cavity. At the same time, *the bottom of the sampling cloth* or *the lower part* is represented by the extremity of the sampling cloth that comprises its *distal end;* that is the point of the sampling cloth opposite to the proximal point, i.e. placed at the furthest distance from the proximal point on the insertion direction. In some embodiments, part of the sampling cloth may protrude outside from the vaginal or rectal cavity. In such a situation, the bottom of the sampling cloth will also comprise such part of the sampling cloth. Taking into account the above definitions, then *lateral sides of the sampling cloth* will be the *left and right extremities,* considering the top and bottom as defined above.

*The upper edge of the sampling cloth* is the edge on the top of the sampling cloth while *the lower edge of the sampling cloth* is the edge situated on the bottom of the sampling cloth. According to the invention, "upper", "up" or "above" will refer to a first point or part situated closer to the top of the sampling cloth relative to a second point of reference, while the second point will be situated "lower", "down" or "below", respectively, to said first point or part.

According to the invention *oriented upwards* means oriented towards the top of the sampling cloth while *oriented downwards* means oriented towards the bottom of the sampling cloth.

The distance from the proximal end to the distal end of the sampling cloth defines *the length of the sampling cloth.*

*The central longitudinal axis of the swab applicator* or *of the sampling cloth* or *of the swab head/rod* or of any part of the swab applicator is defined as an imaginary line passing through the centroid of the cross sections along a longitudinal axis of the swab applicator/sampling cloth/swab head/swab rod etc, comprising its distal end. *The longitudinal axis* passes through the swab applicator or any part of the swab applicator from its proximal end to its distal end as defined above, or preferably passes through the at least part of the swab applicator/sampling cloth/swab head/swab rod etc. containing its distal end.

*The central transverse axis of the swab applicator* or *of the sampling cloth* or *of the swab head/rod* or of any part of the swab applicator is defined as an imaginary line passing through the centroid of the cross sections along a transverse axis of the swab applicator/sampling cloth/swab head/swab rod..etc.

*The transverse axis* passes through the swab applicator or any part of the swab applicator from left to right or from one lateral side to the other lateral side as defined above and it is always perpendicular to the relevant section on the longitudinal axis.

## Brief description of the drawings

Other objectives, advantages and characteristics of the invention shall be presented in the following description of the embodiments, which do not restrict the purpose and extent of this patent application, accompanied by drawings in which:

**Fig. 1** depicts a front view of a sampling cloth according to an embodiment of the present invention;

**Fig. 2** depicts a lateral view of a swab applicator comprising a bent section according to an embodiment of the present invention;

**Fig. 3a** depicts a front view of a swab kit according to an embodiment of the present invention;

**Fig. 3b** depicts a lateral view of a swab kit according to an embodiment of the present invention;

**Figs. 4a and 4b** represent sequential views of some steps of a method for sampling according to the invention, with a swab kit according to an embodiment wherein the sampling cloth is provided with proximal flaps and lateral flaps and is inserted and then rotated clockwise inside a vaginal cavity;

**Figs. 5a-5c** show the embodiment of a swab kit after removal from the bodily cavity and sequential views of some steps of a method for rupturing the sampling cloth on the rupture zone in order to separate the upper zone of the sampling cloth from the lower zone of the sampling cloth;

**Figs. 6a-6d** show the embodiment of a swab kit after rupturing the sampling cloth on the rupture zone and sequential views of the steps for placing the swab applicator together with the free upper zone (or pouch) of the sampling cloth inside a sealable recipient in order to detach the upper zone (or the pouch) of the sampling cloth from the swab head and placing the upper zone (or the pouch) of the sampling cloth within a sealable recipient, and sealing said recipient.

## Detailed description of the invention

### Swab applicator with a bent section

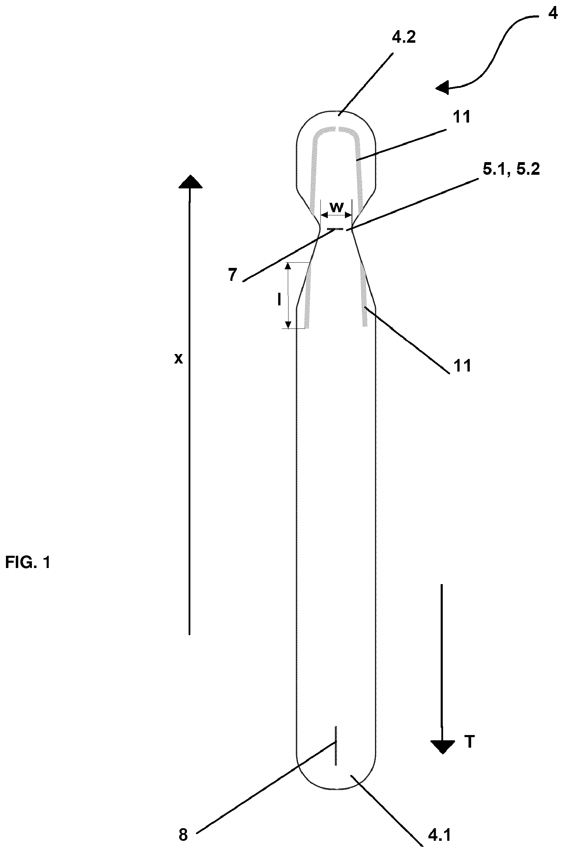

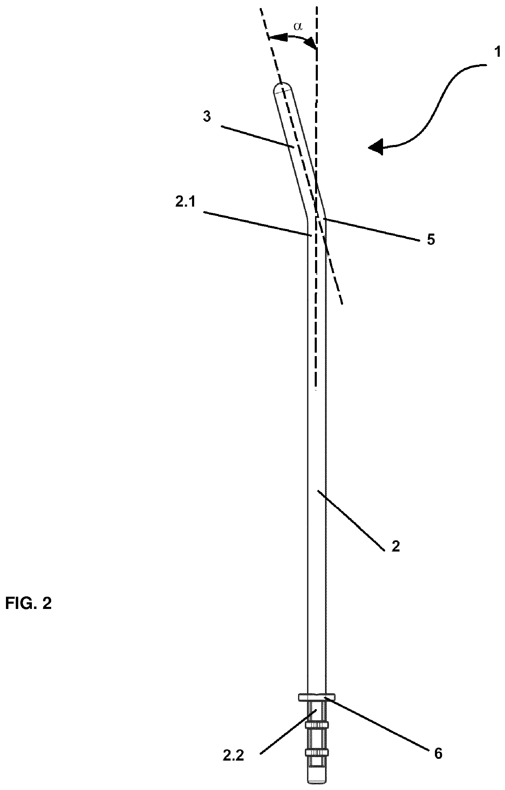

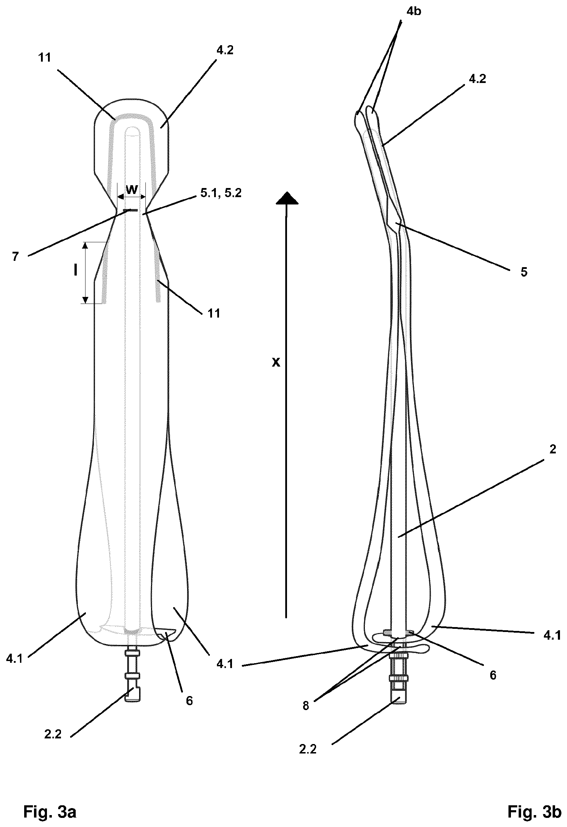

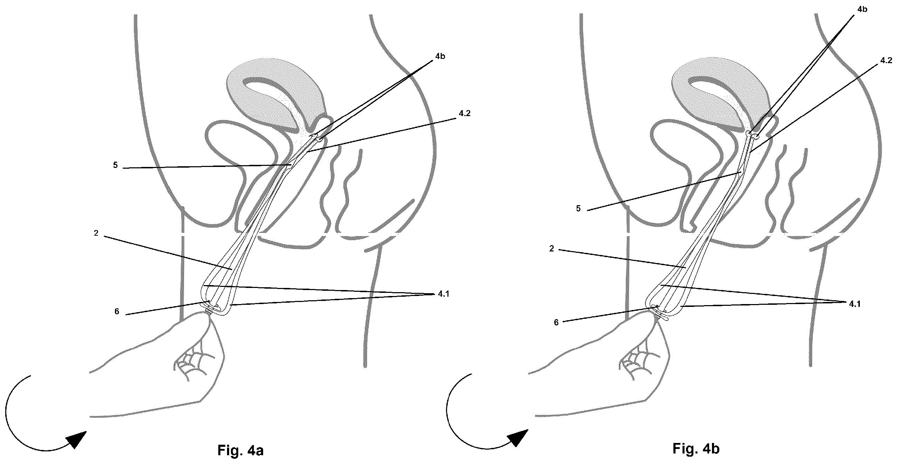

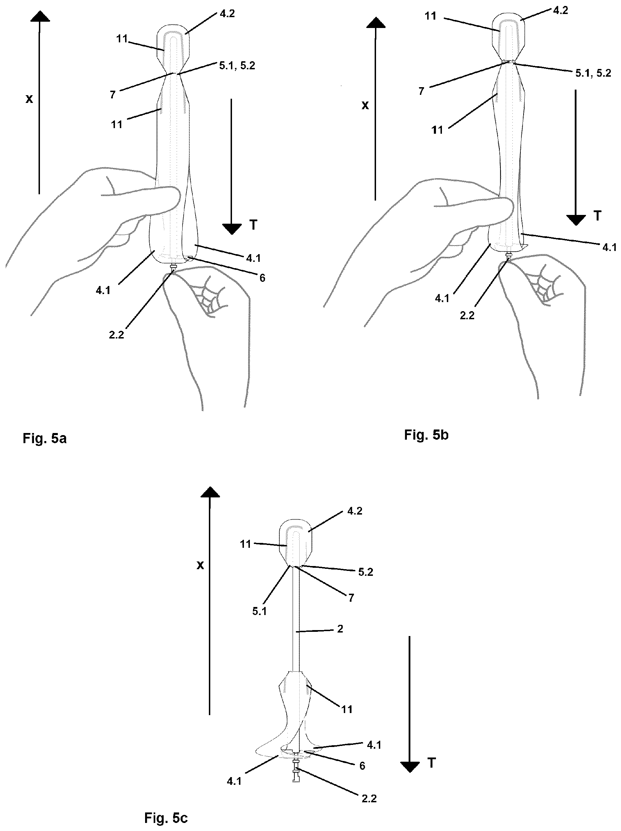

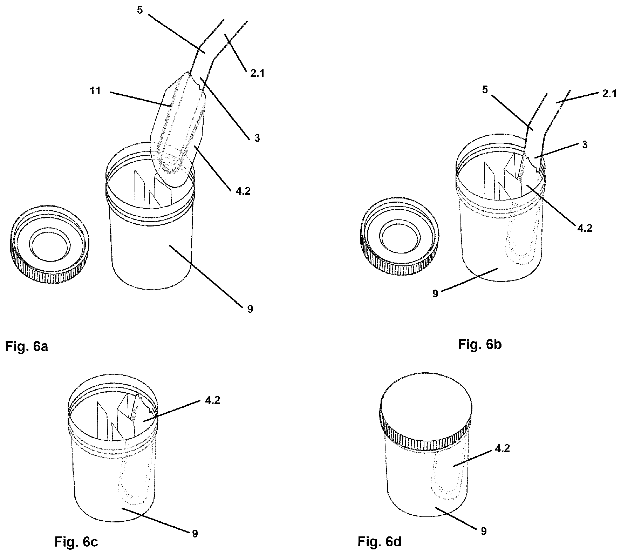

The figures 1 - 6 present a swab applicator (1) of the swab kit according to the present invention for sampling a specimen from a human or animal bodily cavity by use of a flexible sampling cloth (4) as described in claim 1, comprising a longitudinal swab rod (2) and an elongated swab head (3) disposed at a proximal end (2.1) of the swab rod (2). The longitudinal swab rod (2) and the elongated swab head (3) are connected to one another at a bent section (5). The swab rod (2) and the swab head (3) are connected to one another such that their central longitudinal axis corresponds at the bent section (5).

The swab rod (2) and the swab head (3) have a shape, and dimensions adapted to those of the bodily cavity for which the swab applicator (1) is intended and the strength necessary to be able to push the sampling cloth (4) inside the bodily cavity of a human or animal without breaking during insertion and/or removal. The swab applicator (1) may be disposable (one use-only). The swab applicator (1) may be made of any suitable material, such as plastic. Preferably, the swab applicator is made of Polyester (polyethylene terephthalate (PET)), ABS (Acrylonitrile Butadiene Styrene) or low density polyethylene PE-LD, which have low production costs, good flexibility, low weight, recyclable and can be easily molded or thermoformed.

The bent section (5) allows an inclination of the elongated swab head (3) corresponding to a slope percentage measured between the central longitudinal axis of the elongated swab head (3) and the central longitudinal axis of the swab rod (2). The slope corresponds to the inclination of a surface or a line in relation to the horizontal. It can be measured as an angle in degrees, radians or gradians or as a percentage. The slope percentage is calculated using the rise-to-run ratio multiplied by 100 where, "Rise" (or height) is the elevation difference between two points A and B on a plane and "Run" (or width) is the horizontal distance between the two points.

In the context of the present invention, point A is the proximal end (2.1) of the swab rod (2) and point B is the proximal end of the elongated swab head (3). The angle of inclination i.e the slope angle ($\alpha$) is measured between the central longitudinal axis of the elongated swab head (3) and the central longitudinal axis of the swab rod (2). The slope ratio is calculated using the mathematical formula 1, the slope percentage is calculated using formula 2 and the slope angle ($\alpha$) is calculated using formula 3 presented below wherein L is the amount of run, H is the amount of rise and tan$^{-1}$ is the inverse of the trigonometric function tangent of the slope ratio:

$$\text{Slope Ratio} = \frac{\text{Rise}}{\text{Run}} = \frac{H}{L} \qquad (1)$$

$$\text{Slope Percentage} = \frac{H}{L} \times 100 \qquad (2)$$

$$\text{Slope Angle } (\alpha) = \tan^{-1}\left(\frac{H}{L}\right) \qquad (3)$$

In a preferred embodiment of the present invention, the amount of rise (H) of the slope percentage is preferably within a range of about 5 mm to about 10 mm, more preferably about 7 mm to about 10 mm. The amount of run (L) is preferably within a range of about 25 mm to about 30 mm, more preferably about 30 mm. The angle of inclination ($\alpha$) may be of about 9° to about 22° corresponding to a slope percentage of about 16% to about 40%.

The specific inclination of the elongated swab head (3) allows for a precise positioning of the swab applicator (1) for sampling a specimen from a human or animal bodily cavity by use of a sampling cloth without the use of a speculum, for example in the case of a vaginal cavity, and according to the particular position of the women during examination/sampling and of the individual anatomy, without causing discomfort or without damaging the tissue of the sampled organ or accidentaly break the swab head of the swab applicator during insertion into the bodily cavity. More specifically, the vagina is an open cavity in the form of fibro-muscular tube with walls that are easily distensible. The vagina is in the form of a tube having at the extremities an external opening (the vaginal opening) and an internal opening (communicating with the uterus via the cervix). Depending on the position of the woman, the cervix may take different positions relative to the vaginal tube. For example, if a woman is in an upright position, normally the cervix is positioned in the center of the vaginal tube, while if a woman is in a reclining/gynecological position, the cervix is usually eccentric, i.e. closer to the walls of the vaginal tube. When wanting to collect specimens from the cervix, the following problem appears with traditional applicators: because of their straight shape and rigidity, the proximal end of the applicator will be guided by the tubular shape of the vaginal cavity to the center of the internal opening and therefore cannot efficiently contact and/or scrape the cervix when the latter is positioned eccentrically.

According to the present invention, the applicator has an angle of inclination (α) which positions the proximal end of the elongated swab head (3) away from the central longitudinal axis of the longitudinal swab rod (2). This allows that by the rotation of the swab rod, the swab head is also rotated describing an eccentric movement which ensures that the swab head (3) reaches and contacts the cervix for an improved sampling. This is achieved by a simple rotation of the swab rod (2) around its central longitudinal axis, which is simple and less traumatic for the woman. The angle of inclination (α) is chosen taking into account the different angles in which the vaginal cavity and cervix or other parts of various bodily cavities to be examined are placed, depending on the individual anatomy and on variations in the size, shape and orientation of the bodily cavity among patients, or postoperative distortions in anatomy. According to a further example, in postmenopausal women, the squamo-columnar junction recedes making it difficult to obtain good amount of endocervical cells and cells from the transformation zone (TZ). The transformation zone (TZ) is that area of epithelium that lies between the native and unaffected columnar epithelium of the endocervical canal and the native squamous epithelium deriving from the vaginal and ectocervical squamous epithelium. The endocervical canal is lined with glandular epithelium, and the ectocervix is lined with squamous epithelium. The sample taker must aim to sample as much of the TZ as possible as this is the area that pre-cancerous cells develop.

The eccentric rotation of the swab applicator (1) inside the bodily cavity during sampling of the specimens allows for a precise and quantitative sampling of specimens by following for example, the shape of the endocervix, the transformation zone (TZ) and ectocervix as well. Thus, the swab applicator (1) is suitable for every cervix shape. The shape of the swab applicator (1) having a bent section (5) allows also for more safely, efficiently, and reproducibly inserting the applicator into a bodily cavity of a patient.

The bent section (5) may have an arc shape with a radius within a range of about 0.9 mm to about 2.35 mm which facilitates insertion/removal of the swab applicator (1) and avoids damages to the tissue during the sampling process.

A distal end (2.2) on the longitudinal swab rod (2) comprises stopper means (6), preferably mounted in a horizontally protruding manner with respect to a direction of insertion (x) of the sampling cloth or formed thereon in an integrally protruding manner having two protrusions. The stopper means (6) is used for fixing the sampling cloth (4) to the swab applicator (1) during insertion and removal of the swab applicator (1) from the bodily cavity. The stopper means (6) has preferably a structure in which the stopper (6) is easily grasped by a user's hand.

The swab applicator (1) may be releasably inserted, centered or off centre, in a dedicated pocket of the sampling cloth (4). Preferably, the width of said pocket is 2÷6 mm bigger than the diameter of the swab applicator (1), for a smooth insertion and removal. Optionally, the swab applicator (1) may have a gripping area, for gripping during insertion and removal of the swab applicator from the bodily cavity. Said gripping area may have an adherent surface, or may be shaped so that it can be easily and firmly held by the human hand. For example, the swab applicator may have, along the gripping area, a series of recesses or elevations.

The swab rod (2) and the swab head (3) may be made in the form of a solid or hollow body which can be: a cylindrical body, an elongated rectangular prism or any suitable shape preferably with smooth, curved or rounded lateral sides/ends or extremities, preferably chamfered or with a radius within a range of about 0.1x45° to about 0.3x45°, which facilitates insertion/removal of the swab applicator and avoids damages to the tissue during the sampling process. For human use, such a swab applicator (1) may have for example, a length (the height of the applicator) of about 120 mm to about 150 mm and a width (diameter of the applicator) below 10 mm, more preferably below 5 mm.

In all embodiments, the length of the swab applicator (1) is adapted to the bodily cavity in which it is desired to be inserted. Thus, in case it is used for insertion into the human cavity, the length of the swab applicator (1) is preferably maximum 150 mm.

In all embodiments, the width and cross-section of the swab applicator (1) are adapted to the bodily cavity in which it is meant to be inserted and are proportional to the swab applicator's dimensions. The proximal end of the swab applicator (1) which is also the proximal end of the elongated swab head (3) may have a smooth, rounded shape which facilitates insertion of the swab applicator (1) into the bodily cavity and/or into a dedicated pocket of the sampling cloth (4) without damaging/protruding the cloth. Preferably, the diameter of the proximal end of the swab head (3) or swab applicator (1), in a front longitudinal cross-section is within a range of about 1.5 mm to about 2.5 mm.

In a preferred embodiment the maximum diameter of the swab rod and of the swab head is of 2÷3 mm.

The swab applicator (1) is preferably made by injection moulding, which is a manufacturing process for producing parts by injecting molten material into a mould, or mold. Moulds can be of a single cavity or multiple cavities. In multiple cavity moulds, each cavity can be identical and form the same parts/products or can be unique and form multiple different geometries during a single cycle. Preferably, for the manufacturing of the swab applicator (1), a multi-cavity mould is used because we will achieve more products faster such that we will have a shorter lead time per batch, more efficient use of the cycle time and lower product price.

## Sampling cloth

According to another aspect of the present invention, there is provided a sampling cloth (4) of the swab kit according to the present invention for sampling a specimen from a human or animal bodily cavity, wherein the sampling cloth (4) is a low-absorbent, flexible sampling cloth (4) made of a fabric with an absorbancy of 3.5 g/g or less, the sampling cloth (4) having a distal end (4.1) and a proximal end (4.2), said distal and proximal ends (4.1, 4.2) defining an insertion direction (x) inside a human or animal bodily cavity by means of a swab applicator wherein said proximal end (4.2) is the end that first enters the bodily cavity and said distal end (4.1) is the end that last enters the bodily cavity, said sampling cloth (4) further comprising at least one rupture zone positioned between an upper zone and a lower zone of said cloth (4), wherein said upper zone extends from the proximal end (4.2) towards the rupture zone and said lower zone extends from the rupture zone towards the distal end (4.1) of said cloth (4) in an opposite direction of insertion (x) inside a human or animal bodily cavity wherein the sampling cloth (4) is configured to rupture on said at least one rupture zone upon exertion of a tearing force (T) onto said distal end (4.1) of said cloth (4), when the proximal end (4.2) of said cloth (4) is maintained in a fixed position relative to the distal end (4.1), thus separating the upper zone from the lower zone. The sampling cloth (4) of the swab kit of the present invention may also be used for collecting samples for detecting bacteria (such as *Neisseria gonorrhea, Chlamidia trachomatix, Mycolpasma hominis, Mycoplasma urealyticum, Syphilis, Streptococcus),* parasites (such as *Trichomonas vaginalis*) or fungi (such as *Candida albicans).*

The sampling cloth (4) of the swab kit according to the invention is designed to be inserted into the bodily cavity of a human or animal, by means of a swab applicator. The swab applicator as described above, allows an even more efficient sampling of the cells and manipulation without contaminating the sample. The sampling cloth (4) of the swab kit according to the invention is made of a material suitable to be introduced into a bodily cavity such as the vagina or rectum. Thus, the sampling cloth (4) can be comfortably fitted inside the bodily cavity. Said suitable material will be flexible, by which it is to be understood a material that will bend and unbend to follow the shape of for example, the vagina or rectum. Also, a suitable material will be preferably atraumatic, by which it is to be understood a material that may be put into contact with or made to slide over or wipe a surface of a body membrane such as the vaginal or rectal mucosa without causing any injury or discomfort such as irritation, pain etc., preferably a fabric with a soft carded smooth surface. The use of a sampling cloth (4) made of such a flexible, atraumatic material has the advantage that avoids the irritation of the bodily cavity, pain and discomfort during the insertion that are usually associated with the use of the swab kits from the state of the art. Preferably, when the sampling cloth (4) is introduced into the vagina with the aid of the swab applicator (1), the sampling cloth (4) above and around said applicator (1) will protect the vaginal mucosa from direct contact with the hard material of the swab applicator (1). According to the present invention, the sampling cloth (4) covers the swab applicator (1) at least partially and in a preferred embodiment, the elongated swab head (3) of the swab applicator (1) is completely covered by the proximal end (4.2) of the cloth (4) during insertion and retraction of the swab applicator (1) into and out of the bodily cavity and the bent section (5) and the longitudinal swab rod (2) are partially covered by the cloth (4) during insertion and retraction of the swab applicator (1) into and out of the bodily cavity.
The sampling cloth (4) can be made of at least one sheet of fabric having any suitable shape; preferably it may have, for example, a substantially rectangular, square, trapezoidal, parallelepipedic, oval or circular shape.

Advantageously, a suitable material for the sampling cloth (4) of the swab kit according to the invention is a material that can catch specimens from the bodily cavity and preferably retain said specimens on its surface, while at the same time being able to subsequently easily release said specimens so that they can be analyzed. Therefore, the sampling cloth (4) has advantageously a surface coming into contact with the bodily cavity, but with little or no absorbency, so that only a small amount of the specimens are absorbed into the fabric of the sampling cloth (4). According to the invention, the absorbency is less than 3.5 g/g, preferably less than 3 g/g, more preferably less than 2 g/g measured using the Syngina protocol for measuring the absorbency of tampons. For example, an absorbency of 3.5 g/g means that 3.5 grams of liquid are absorbed per 1 gram of material.

Preferably, the sampling cloth (4) is made of a fabric having low thickness. A sampling cloth having low thickness will be able to catch the specimens on its surface, but it will not, or only minimally, transfer, collect and retain them in its depth. Such a sampling cloth has the advantage that, unlike a sponge or a tampon, it will not retain inside the collected specimens (cells, proteins, DNA, etc.) during the analysis and evaluation steps. Instead, the sampling cloth (4) will easily liberate the specimens from the sampling cloth, so the final quantity of specimens to be analyzed is maximized, which raises the precision rate (accuracy) of the sampling test. The thickness of the sampling cloth (4) should be of 1 mm or less, preferably 0.4 mm or less, more preferably 0.2 mm or less, which means a thickness much smaller compared to a normal tampon.

The material of the sampling cloth, suitable to be comfortably inserted into a bodily cavity, may be chosen from the group of a woven or non-woven textile, for example made of synthetic fibers (such as polyester, polypropylene, polyethylene, polyamide, polyacetate, polyvinyl acetate), semi-synthetic fibers (such as viscose, modal, lyocell), plant fibers (such as cotton), animal fibers (such as silk), or combinations thereof. In a preferred embodiment, the material is biodegradable, thus reducing the impact on the environment. Preferably, the material is a non-woven textile made of synthetic fibers, since such materials are atraumatic and have the desired low absorbency. Also, such products may have low production costs.

In a preferred embodiment, the material is also thermo-fusible, so that it may be welded to each other to the lateral sides and/or top of the sampling cloth (4) so that the sampling cloth (4) can be removably attached to the elongated swab head (3). Welding is a fabrication process that joins materials, by using high heat to melt the parts together and allowing them to cool, causing fusion. In a preferred embodiment, only the lateral sides of the sampling cloth (4) are welded to each other and the top side of the sampling cloth (4) may be folded and fixed to be able to withstand for example a tearing force (T) who might rupture the top side. In a more preferred embodiment of the present invention, the upper zone of the sampling cloth (4) comprises two sheets of fabric, each having a proximal margin and a distal margin. The two sheets are fastened to each other by fastening means (11) such as continuous welding lines, sewing lines, and/or glue lines to form a pouch having an open end, a short side opposed to the open end and situated toward the proximal margin, and two lateral sides situated along said proximal margin; the short side of the pouch and the two lateral sides are situated at a distance of at least 1 mm, preferably about 1 mm to 8 mm, more preferably 1 mm to 5 mm, or 1 mm to 3 mm from the proximal margins of the sheets, forming two proximal flaps (4b) fixed to each other by the fastening means (11) at the short side and at the lateral sides and having the opposite end free. The two proximal flaps (4b) can transition from a closed state, wherein their two free margins touch or are very close to each other, to an open state, wherein their two free margins get away from each other. Each proximal flap (4b) has an inner face, which is the surface of the flap positioned toward the other flap when in closed state, and an opposite outer face, which is the surface of the flaps positioned toward the exterior of the sampling cloth (4) when in closed state. A sampling cloth (4) provided with such proximal flaps (4b) can efficiently sample specimens, because, during insertion of the sampling cloth (4) into the bodily cavity, the proximal flaps (4b) transition to said open state, by the friction with the walls of the bodily cavity. This allows the specimens to be collected mainly on the inner faces, which are often positioned in the bodily cavity, when the cloth (4) is fully inserted, at the most important area for sampling, which is usually in the vicinity of the internal opening of the bodily cavity. During removal from the bodily cavity, the proximal flaps (4b) transition back to the closed state, thus catching the specimens between the flaps, on their inner faces. This ensures that a sufficient amount of specimens are successfully sampled and kept on the inner faces of the flaps (4b), which are not lost because only the external faces and rest of the sampling cloth (4) are wiped due to friction with the walls of the bodily cavity during removal.

In a more preferred embodiment, the sampling cloth (4) is made of a non woven fabric with a soft carded smooth surface comprising a non-woven polyethylene/-polyester bicomponent.

In a preferred embodiment, the sampling cloth (4) according to the invention may have a region having a rougher surface than the rest of the sampling cloth (such as more roughly checkered organic cotton, linen or burlap), preferably placed so that it is most likely to reach and, by scratching the tissue, collect samples from a targeted area of the bodily cavity. For example, in order to collect samples from the cervical os, the rougher fabric can be placed at the top (i.e. proximal end (4.2)) of the sampling cloth (4). The rougher fabric may, for example, be attached to the fabric of the sampling cloth by any suitable means such as gluing, welding, sewing, etc, or it may be integral part of the sampling cloth. A sampling cloth (4) having such a region with a rougher surface will be able to collect, in less time, specimens from the targeted area.

The sampling cloth (4) of the swab kit according to the invention is suitable to be inserted into the vaginal or rectal cavity of a human or animal. Thus, the person skilled in the art will understand to choose the dimensions of the sampling cloth (4) adapted to the dimensions of the bodily cavity where it is to be inserted.

The vagina and rectum are open cavities in the form of fibro-muscular tubes with walls that are easily distensible. The vagina is in the form of a tube having at the extremities an external opening (the vaginal opening) and an internal opening (communicating with the uterus). The rectum is in the form of a tube having at the extremities an external opening (the anus) and an internal opening (communicating with the large intestine); near the external opening it has a dilated portion, the rectal ampulla, where the sampling cloth (4) according to the invention is meant to reach for sampling. The external openings of the vagina and rectum are substantially circular. The width (diameter) of the tubes (vagina and rectum) varies throughout their length, with the minimum width being at the external opening of the bodily cavity. For example, the human adult vagina or rectum at rest has, at the external opening, a width of about 2.5 cm.

Whatever the shape of the sampling cloth when outside the bodily cavity, due to the fact that it is made of a flexible fabric, when inserted into the bodily cavity by pushing it through the substantially circular external opening, the sampling cloth will collapse, e.g. deform, fold, strangle and/or twist to pass through the external opening and then will unfold to roughly follow the shape of the cavity. Therefore, the dimensions of the sampling cloth for inserting into the human vaginal or rectal cavities will be chosen such that, in said collapsed position, the maximum width of the sampling cloth will be of less than 2.5 cm, so that it can be comfortably inserted into the bodily cavity.

For example, the length of the human adult vagina at rest varies from about 5 to about 14 cm, and the length of the human adult rectal ampulla varies from about 4 to about 6 cm. The skilled person will preferably choose the dimensions of the sampling cloth (4) for completely inserting in the vagina of a human so that, when inserted, the sampling cloth will have the length approximately equal or slightly less than the human vagina so that it can collect specimens from all parts of the vagina. Therefore, the skilled person will choose in this case a sampling cloth with a length of less than 14 cm, such as about 12 cm or about 10 cm or about 8 cm or less. For insertion in the human rectum the sampling cloth will be smaller, in order to stay in the rectal ampulla, for example having a length of less than 5 cm, preferably less than 3.5 cm.

When inserted into the bodily cavity, which means after the sampling cloth (4) has been pushed inside the bodily cavity by means of the swab applicator (1), the sampling cloth (4) will preferably take a shape having the proximal end (4.2) closest to the internal opening of the bodily cavity and the distal end (4.1) closest to the external opening of the bodily cavity, and will occupy a roughly tubular space. Thus, the skilled person will be able to shape/choose the dimensions of the sampling cloth (4) so that when inserted into the bodily cavity, the sampling cloth (4) will preferably have a length for example up to the maximum length of the vagina or of the rectal ampulla.

During immune-cytochemical tests and HPV testing for example, the sampled specimens must be capped/sealed tightly to be safely transported and to avoid leakage and/or potential contamination of specimens. The sealable recipient, such as a vial is used for storing and transporting the sampling cloth (4), after removal from the bodily cavity, to a diagnosis facility such as a laboratory. The sealable recipient or vial is called a specimen bottle (container) or sampling bottle and comprises a conical-bottom, a threaded cap and a preservative solution, such as 50% ethanol based solution. The shape and height of the specimen bottle is standardized. Usually the shape of the specimen bottle is cylindrical having a diameter under 30 mm. The height of the specimen bottle varies according to the specific specimen type/source, for example 50 mm or more. Optionally, the specimen bottle may have an interior rim and at least a separate opening. The separate opening is usually used for depositing the collected sample of specimen and the other opening for automatic handling of the specimen bottle during analysis. Appropriate storage and handling are necessary to maintain the integrity of the specimen and, consequently, the test results.

When using a known swab applicator, the swab head must be detached from the applicator to be deposited inside the specimen bottle together with the sampled specimens. This is due to the smaller dimensions of the specimen bottle compared to the length of the swab applicator. The heads of the known swab applicators are never removed from the preservative vial containing the collected sample. The swab head can damage the collected cells or specimen inside the vial during transport because the weight of the swab head can crush the sampling cloth with the collected sample between the walls of the vial, thus physically breaking apart the cell walls. This can affect the quality of the analysis in a negative way and also the time for preparation of the collected sample will be increased. Also, the way of separation of the swab head from the swab applicator can contaminate the collected sample.

The present invention has overcome the above mentioned problems by providing the swab kit which comprises a sampling cloth (4) with at least one rupture zone.

The sampling cloth (4) of the swab kit according to the invention comprises also an upper zone and a lower zone. The upper zone extends from the proximal end (4.2) of the cloth (4) towards the rupture zone and the lower zone extends from the rupture zone towards the distal end (4.1) of the cloth (4) when viewed in an opposite direction of insertion (x) inside a human or animal bodily cavity. The at least one rupture zone is positioned between the upper zone and the lower zone of the sampling cloth (4).

The upper zone of the cloth (4) is removably attached to the swab applicator (1) and the lower zone of the cloth (4) comprises fixing means (8) for fixing the sampling cloth (4) to the swab applicator (1) during insertion and removal of the applicator (1) into and out of the bodily cavity. The fixing means (8) are preferably through holes or slits.

The sampling cloth (4) is configured to rupture on the at least one rupture zone upon exertion of a tearing force (T) onto the distal end (4.1) of the cloth (4), when the proximal end (4.2) of the cloth (4) is maintained in a fixed position relative to the distal end (4.1), thus separating the upper zone from the lower zone. The tearing force (T) is exerted after removal of the swab applicator (1) comprising the sampling cloth (4) from the bodily cavity, by the qualified medical personnel. The free/detached/removed upper zone of the sampling cloth (4) is then placed into a specimen bottle which is then capped, labeled, and sent with appropriate paperwork to the laboratory for processing. Thus, the swab head (3) of the swab applicator (1) of the swab kit according to the invention does not need to be placed inside the specimen bottle during transport, reducing the weight of the probe which will only consist of the upper zone of the sampling cloth of the swab kit. This reduces the risk of damaging the cells during manipulation and transport. If the swab head was placed inside the specimen bottle together with the upper zone, the additional weight of the swab head would increase the impact of the upper zone with the walls of the specimen bottle thus crushing the sampled cells.

The proximal end (4.2) of the cloth (4) is maintained in a fixed position relative to its distal end (4.1), for example, by keeping the swab applicator (1) with the sampling cloth (4) in a substantially upright position (i.e.

straight up). The elongated swab head (3) is completely covered by the proximal end (4.2) of the cloth (4) during insertion and retraction of the swab applicator (1) into and out of the bodily cavity. The proximal end (4.2) of the cloth (4) may have a greater thickness than the remaining material of the sampling cloth (4) or it may be folded and fixed in order to avoid accidental rupture of the proximal end (4.2) during exertion of the tearing force (T).

Another preferred way of maintaining the proximal end (4.2) of the cloth (4) in a fixed position relative to its distal end (4.1) is to position and press the swab applicator (1) with the sampling cloth (4) in a substantially downright position (i.e. straight down) inside the specimen bottle and with the proximal end (4.2) contacting the bottom of the bottle. In this position it is ensured that the collected cells on an area other than the proximal end (4.2) of the cloth (4) remain intact.

In a preferred embodiment, the sampling cloth (4) has an average basis weight (mass per unit area), measured with WSP 130.1 Test method, of around 59.20 g/m2 or less. In another preferred embodiment freely combinable with the previous ones, the average tensile strength MD, representing the force per unit width which is required to rupture a sample orientated in the machine direction, measured with a Test method following WSP 110.4 using a sample width of 25.4 mm (1 inch), a clamp distance of 127 mm (5 inch) and a speed of 500 mm/min (19.7 inch/min), is of around 1924 N/m (48.86 N/inch) and the average elongation at F-max MD, representing the relative increase in length at the maximum force applied on a sample orientated in the machine direction, measured with a Test method following WSP 110.4 using a sample width of 25.4 mm (1 inch), a clamp distance of 127 mm (5 inch) and a speed of 500 mm/min (19.7 inch/min), is of around 38.40 %. In another preferred embodiment, the upper zone of the sampling cloth obtained after rupture has a weight of about 0.1g or less. When the upper zone is placed inside a specimen bottle, due to its very light weight and flexible material, it is less susceptible to shocks resulted from the impact with the bottle's walls. This avoids crushing of the cells collected on the material during transport or manipulation.

Preferably, the at least one rupture zone has a surface area which narrows towards its central transverse axis or towards the central transverse axis of the bent section (5) resulting at least one frangible wall (5.1, 5.2) having a pre-determined minimum width (w) proximally its central transverse axis or proximally to the central transverse axis of the bent section (5). In a preferred embodiment, the bent section (5) is positioned proximally with respect to the upper zone of the sampling cloth (4). The pre-determined minimum width (w) of a frangible wall (5.1, 5.2) may be within a range of about 9 mm to about 15 mm.

In a preferred embodiment, the flexible material of the rupture zone comprises at least one opening (7), preferably a through hole or a slit, having any suitable shape which is configured to rupture upon exertion of the tearing force (T) onto the distal end (4.1) of the cloth (4). Preferably, the at least one opening (7) is positioned centrally on the rupture zone. In a more preferred embodiment of the present invention, the flexible material of the rupture zone comprises at least one opening (7), preferably a plurality of perforations configured to rupture at an area between the perforations. Rupturing the area between each of the individual perforations, directs the tearing energy to the rupture zone to control the rupture in that area. Preferably, the perforations are arranged in an array with each of the perforations spaced equidistantly to define an area that rupture upon exerting a tearing force as defined above. The array can also be defined in a substantially straight line, circular, elliptic or other shape that provides further control of tearing within the rupture zone. The number of perforations may vary and can be based on the shape of the array in which the perforations are arranged. More precisely, the number of perforations depends on the area between every single perforation, the size of the rupture zone as well as the shape of the array, which allows the best absorption of tearing energy to be optimized.

In another preferred embodiment, the flexible material of the rupture zone has a lesser thickness compared to the material's thickness of the upper and/or lower zones. Preferably, the flexible material of the at least one rupture zone may be loosely woven compared to the material of the upper and/or lower zones, or any combination of at least one opening (7) in the material of the rupture zone, lesser thickness and/or loosely woven material may be used.

The tearing force (T) is preferably exerted in an opposite direction to the insertion direction (x) of the swab applicator (1) or in a direction substantially perpendicular to the insertion direction (x) of the swab applicator (1) and away from the central longitudinal axis of the swab applicator (1) (situation not shown in the figures). The skilled person will be able to calculate the appropriate resistance to rupture of the at least one rupture zone proximally to the central transverse axis of the rupture zone by using the common general knowledge in the field of invention, e.g. taking into consideration the width and thickness of the frangible walls (5.1, 5.2), etc. The tearing force (T) can be a lateral or linear force. In this way, it is ensured that the rupture will take place only in the rupture zone and not in other areas of the sampling cloth (4).

**Swab kit**

The swab kit according to the invention comprises a swab applicator (1) and a sampling cloth (4) designed to be inserted into the bodily cavity, as described above. Optionally, the swab kit may comprise a sealable recipient (9) such as a vial, also known as specimen bottle or container for storing and transporting the sampling cloth (4) to a diagnosis facility such as a laboratory. The swab kit may further comprise instructions for its use and/or labeling means. The use of the sampling cloth (4) together with the swab applicator (1) according to the present invention is much simpler and less traumatic. The entire experience is physically and mentally comfortable, while at the same time rendering high quality results. Additionally, by getting into contact with virtually the entire surface of the bodily cavity, as well as the external tissues, every potential specimen of interest (such as cells, nucleic acids, proteins, viruses, bacteria, parasites or fungi) may be caught by the sampling cloth (4) and released when needed.

In a preferred embodiment of the present invention (Figs. 1, 3a-3b, 4a-4b, 5a-5c), the sampling cloth (4) of the swab kit is substantially in the form of two sheets of fabric, each having a proximal margin situated at the proximal end of the swab head (3), a distal margin situated at the distal end (2.2) of the longitudinal swab rod (2), and two lateral margins. It is understood that a cloth as described below can be used also with any type of applicator. Embodiments wherein the swab kit comprises two sheets of fabric are preferred because the method of producing the device from two sheets of fabric is simple and fast. These two sheets of fabric are preferably welded together, preferably on top and lateral sides of the sheets of fabric or the top side is folded and fixed and the lateral sides are welded to each other such that the surfaces of both sheets of fabric will come into direct contact with the bodily cavity, thus maximizing the contact surface where specimens are collected. The two sheets of fabric are preferably fastened to each other by welding or other fastening means (11) such as continuous welding lines, spot welding rows, sewing lines, and/or glue lines, to form a pouch with an open end, preferably positioned proximally with respect to the bent section (5). The pouch comprises also a short side, opposed to the open end and situated toward the proximal end of the swab head (3) and two lateral sides situated along the proximal end of the swab head (3). Preferably, the short side of the pouch and the two lateral sides are situated at a distance of at least 1 mm, preferably of about 1 mm to 8 mm, more preferably 1 mm to 5 mm, or 1 mm to 3 mm from the proximal margins of the two sheets of fabric, forming two proximal flaps (4b) fixed to each other by the fastening means (11) at the short side and at the lateral sides and having the opposite end free. In preferred embodiments, the fastening means (11), at the short side made of two or more rows and/or lines of welding is more resistant to the force of pushing of the swab applicator (1). Also, the fastening means (11) can indicate an insertion direction (x) into the bodily cavity by defining the top and lateral interior walls of the sampling cloth (4) between which the swab applicator (1) can be introduced.

The two proximal flaps (4b) represent the sections of the two sheets of fabric extending from the fastening means (11), such as the short side of the pouch, to the proximal margins of each sheet. The two flaps (4b) can be symmetrical, meaning that they have similar shapes and dimensions, or asymmetrical, meaning they can have shapes and dimensions different from each other. As mentioned, the two proximal flaps (4b) are fixed to each other by the fastening means (11) along the short side of the pouch and at the lateral sides, and have each a free margin opposite the short side and the lateral sides of the pouch that is also the proximal/lateral margin of the respective sheet of fabric. The two proximal flaps (4b) can transition from a closed state, wherein their two free margins touch or are very close to each other, to an open state, wherein their two free margins get away from each other. Each proximal flap (4b) has an inner face, which is the surface of the flap positioned toward the other flap when in closed state, and an opposite outer face, which is the surface of the flaps positioned toward the exterior of the sampling cloth (4) when in closed state. A sampling cloth (4) provided with such proximal flaps (4b) can efficiently sample specimens, because, during insertion of the swab kit into the bodily cavity, the proximal flaps (4b) transition to said open state, by the friction with the walls of the bodily cavity. This allows the specimens to be collected mainly on the inner faces, which are often positioned in the bodily cavity, when the swab kit is fully inserted, at the most important area for sampling, which is usually in the vicinity of the internal opening of the bodily cavity. During removal from the bodily cavity, the proximal flaps (4b) transition back to the closed state, thus catching the specimens between the flaps, on their inner faces. This ensures that a sufficient amount of specimens are successfully sampled and kept on the inner faces of the flaps (4b), which are not lost because only the external faces and rest of the sampling cloth (4) are wiped due to friction with the walls of the bodily cavity during removal.

Each surface area of the two sheets of fabric of the sampling cloth (4) narrows in the proximity of at least one rupture zone, as described above, towards its central transverse axis or towards the central transverse axis of the bent section (5) resulting two frangible walls (5.1, 5.2) having a pre-determined minimum width (w) of each of the two sheets of fabric proximally to its central transverse axis or proximal to the central transverse axis of the bent section (5). The pre-determined minimum width (w) is preferably within a range of about 9 mm to about 15 mm, more preferably about 9 mm. Each frangible wall (5.1, 5.2) further comprises at least an opening (7) as described above, situated in the centre of the at least one rupture zone.

The two sheets of fabric are further fastened to each other by the fastening means (11) along a proximal end of the longitudinal swab rod (2) on a pre-determined minimum length (I), preferably about 15 mm to about 25 mm and unfastened from each other towards a distal end of the longitudinal swab rod (2). Preferably, the bent section (5) and the longitudinal swab rod (2) are partially covered by the sampling cloth (4) during insertion and retraction of the swab applicator (1) into and out of the bodily cavity.

Each distal end (4.1) of the two sheets of fabric may have a through hole (8) configured to allow attachment of each of the distal end (4.1) of the two sheets of fabric to the stopper means (6) during normal use of the swab applicator (1) and detachment of each of the distal end (4.1) of the two sheets of fabric from the stopper means (6) during analysis and evaluation of the specimens.

The stopper means (6) is mounted proximally on the distal end (2.2) of the longitudinal swab rod (2) in a horizontally protruding manner with respect to the insertion direction (x) or is formed thereon in an integrally protruding manner having two protrusions that are configured to alternately penetrate through each of the two through holes (8) of each of the distal end (4.1) of the two sheets of fabric.

The frangible walls (5.1, 5.2) are strong enough to hold together the pouch and the two sheets of fabric during normal use of the swab applicator (1) and to rupture upon exertion of a tearing force (T) onto the distal end (4.1) of the cloth (4), in a direction opposite to the insertion direction (x) of the swab applicator (1), when the proximal end (4.2) of the cloth (4) is maintained in a fixed position relative to the distal end (4.1), thus separating the pouch from the two sheets of fabric. Preferably, the pouch is made of the flexible fabric described above having a total basis-weight of about 0.05 gx2. Such a pouch will also allow for an easier release of the collected specimens (cells, proteins, DNA, etc.) from the sampling cloth (4) during the analysis and evaluation steps. Also, due to its very light weight and flexible material, the collected cells/specimen will not be crushed between the pouch and the walls of the specimen bottle during transport or during a shock.

As mentioned before, the proximal end of the two sheets of fabric may be folded and fixed resulting a region having a rougher surface and/or thicker surface than the rest of the sampling cloth (4) to collect, in less time, specimens from the targeted area and to prevent an accidental rupture of the proximal end of the cloth (4) upon exertion of the tearing force (T). The lateral sides are welded to each other such that the surfaces of both sheets of fabric can form the proximal lateral flaps (4b) as described above.

The sampling cloth (4) according to the present embodiment may be used together with an applicator having a bent section (5) as described above or with any applicator (not covered by the present invention) for insertion of a sampling cloth in a human or animal bodily cavity.

Yet in a preferred embodiment, the sampling cloth (4) of the swab kit has all the features and characteristics described throughout the present description.

The sterilization of the sampling cloth (4) is preferably made using UV light.

In an alternative embodiment of the invention, the sampling cloth (4) as described above can be used with any type of conventional swab applicator.

The recipient (9) of the swab kit is preferably made of a suitable standard material such as plastic, and has the dimensions adapted to house the entire upper zone, pouch of the sampling cloth (4) or sampling cloth (4). Said recipient (9) is provided with at least one opening equipped with sealing means. At least one of the openings of said recipient (9) has dimensions sufficient to allow the user of the kit to easily place the upper zone or pouch of the sampling cloth (4) inside the recipient (9), preferably without squeezing the cloth (4), which might result in removing part of the collected specimens or crushing the walls of the collected cells. Therefore, the dimensions of said opening will depend on the width of the sampling cloth (4). For example, said recipient (9) may be in the form of a plastic jar, vial or tube provided with a thread cap.

Optionally, said recipient (9) may contain means for preserving the specimen or further preparing it for a desired subsequent examination, such as saline water, culture medium (for bacterial analysis), KOH solution (for fungal analysis), etc.

Although the invention has been described in connection with particular illustrative embodiments, it will be clear that it is not, in any way, limited to these embodiments and that it covers all the technical equivalents of the means described and their combinations, insofar as the same function is achieved. The scope of the invention is defined by the appended claims solely.

## Claims

1. Swab kit comprising:

    a swab applicator (1) for sampling a specimen from a human or animal bodily cavity by use of a low-absorbent flexible sampling cloth (4) comprising:

      - a longitudinal swab rod (2) and
      - an elongated swab head (3) disposed at a proximal end (2.1) of said swab rod (2)

    wherein said swab applicator (1) is at least partially covered with said low-absorbent, flexible sampling cloth (4) and used to insert and remove the sampling cloth (4) into and out of the bodily cavity,
    the sampling cloth (4) having a distal end (4.1) and a proximal end (4.2), said distal and proximal ends (4.1, 4.2) defining an insertion direction (x) inside a human or animal bodily cavity by means of the swab applicator (1) wherein said proximal end (4.2) is the end that first enters the bodily cavity and said distal end (4.1) is the end that last enters the bodily cavity, said sampling cloth (4) further comprising at least one rupture zone positioned between an upper zone and a lower zone of said cloth (4), wherein said upper zone extends from the proximal end (4.2) towards the rupture zone and said lower zone extends from the rupture zone towards the distal end (4.1) of said cloth (4) in an opposite direction of insertion (x) inside a human or animal bodily cavity and said upper zone of the cloth (4) is removably attached to said swab applicator (1) and the sampling cloth (4) is configured to rupture on said at least one rupture zone upon exertion of a tearing force (T) onto said distal end (4.1) of said cloth (4), when the proximal end (4.2) of said cloth (4) is maintained in a fixed position relative to the distal end (4.1), thus separating the upper zone from the lower zone **characterized in that**

the longitudinal swab rod (2) and the elongated swab head (3) are connected to one another at a bent section (5) wherein the bent section (5) allows an inclination of the elongated swab head (3) measured between the central longitudinal axis of said elongated swab head (3) and the central longitudinal axis of said swab rod (2) and **in that** stopper means (6) is disposed in the proximity of a distal end (2.2) on the longitudinal swab rod (2) for fixing the sampling cloth (4) to said swab applicator (1) during insertion and removal of the applicator (1) from the bodily cavity wherein the sampling cloth (4) is made of a fabric with an absorbancy of 3.5 g/g or less, and said lower zone of the cloth (4) comprises fixing means (8) for fixing the sampling cloth (4) to said swab applicator (1) during insertion and removal of the applicator (1) into and out of the bodily cavity.

2. Swab kit according to claim 1 wherein said inclination of the elongated swab head (3) corresponds to a slope percentage of about 16% to about 40%.

3. Swab kit according to claim 2 wherein an amount of rise (H) of said slope percentage is within a range of about 5 mm to about 10 mm.

4. Swab kit according to claim 2 or 3 wherein an amount of run (L) of said slope percentage is within a range of about 25 mm to about 30 mm, more preferably about 30 mm.

5. Swab kit according to any of the preceding claims wherein an angle of inclination ($\alpha$) measured between the central longitudinal axis of said elongated swab head (3) and the central longitudinal axis of said swab rod (2) is of about 9° to about 22°.

6. Swab kit according to any of the preceding claims wherein said bent section (5) has preferably an arc shape with a radius within a range of about 0.9 mm to about 2.35 mm.

7. Swab kit according to any of the preceding claims wherein the swab applicator (1) is made of a plastic material, preferably polyester (polyethylene terephthalate (PET)) or ABS (Acrylonitrile Butadiene Styrene).

8. Swab kit according to any of the preceding claims wherein said sampling cloth (4) is made of the fabric having a thickness of 1 mm or less, preferably 0.4 mm or less, most preferably 0.2 mm or less.

9. Swab kit according to any of the preceding claims wherein said sampling cloth (4) is made of the fabric having a basis-weight of around 59.20 g/m2 or less.

10. Swab kit according to any of the preceding claims wherein said sampling cloth's (4) upper zone is made of the fabric having a weight of 0.1g or less.

11. Swab kit according to any of the preceding claims wherein the upper zone of said sampling cloth (4) comprises:

    two sheets of fabric, each having:

    - a proximal margin;
    - a distal margin,

    wherein said sheets are fastened to each other by fastening means (11) such as continuous welding lines, sewing lines, and/or glue lines to form a pouch having an open end, a short side opposed to the open end and situated toward the proximal margin, and two lateral sides situated along said proximal margin, wherein said short side of the pouch and said two lateral sides are situated at a distance of at least 1 mm, preferably about 1 mm to 8 mm, more preferably 1 mm to 5 mm, or 1 mm to 3 mm from the proximal margins of said sheets, forming two proximal flaps (4b) fixed to each other by the fastening means (11) at the short side and at the lateral sides and having the opposite end free.

12. Swab kit according to any of the preceding claims wherein the at least one rupture zone of said sampling cloth (4) has a surface area which narrows towards its central transverse axis resulting at least one frangible wall (5.1, 5.2) having a pre-determined minimum width (w) proximally to the central transverse axis of the at least one rupture zone.

13. Swab kit according to any of the preceding claims wherein the flexible material of the at least one rupture zone of said sampling cloth (4) comprises at least one opening (7), preferably a through hole or a slit or a plurality of perforations or wherein the at least one rupture zone has a lesser thickness compared to the material's thickness of the upper and/or lower zones or wherein the at least one rupture zone is loosely woven compared to the material of the upper and/or lower zones.

14. Swab kit according to any of the preceding claims configured such that said tearing force (T) is a lateral or linear force exerted in an opposite direction to the insertion direction (x) of the swab applicator or in a direction substantially perpendicular to the insertion direction (x) of the swab applicator and away from the central longitudinal axis of said swab applicator, after removal of the swab applicator (1) comprising the sampling cloth (4) from the human or animal bodily cavity.

15. Swab kit according to any of the previous claims wherein said elongated swab head (3) of the swab applicator (1) is completely covered by said proximal end (4.2) of the sampling cloth (4) during insertion and removal of the swab applicator (1) into and out of the bodily cavity and said bent section (5) and said longitudinal swab rod (2) are partially covered by said sampling cloth (4) during insertion and removal of the swab applicator (1) into and out of the bodily cavity.

16. Swab kit according to any of the preceding claims wherein the bent section (5) is positioned proximally with respect to said upper zone of the sampling cloth (4).

17. Swab kit according to any of the preceding claims wherein said sampling cloth (4) comprises:

- two sheets of fabric, each having:

    - a proximal margin situated at a proximal end of the swab head (3),
    - a distal margin situated at a distal end (2.2) of the longitudinal swab rod (2), wherein said sheets are fastened to each other by fastening means (11) such as continuous welding lines, sewing lines, and/or glue lines to form a pouch having an open end situated proximally with respect to the bent section (5), a short side opposed to the open end and situated toward the proximal end of the swab head (3), and two lateral sides situated along said proximal end of the swab head (3), wherein said short side of the pouch and said two lateral sides are situated at a distance of at least 1 mm, preferably of about 1 mm to 8 mm, more preferably 1 mm to 5 mm, or 1 mm to 3 mm from the proximal margins of said sheets, forming two proximal flaps (4b) fixed to each other by the fastening means (11) at the short side and at the lateral sides and having the opposite end free;

each surface area of the two sheets of fabric narrows in the proximity of at least one rupture zone towards the central transverse axis of the rupture zone resulting two frangible walls (5.1, 5.2) having a pre-determined minimum width (w) of each of the two sheets of fabric proximally to the central transverse axis of the rupture zone; said two sheets of fabric are further fastened to each other by the fastening means (11) along a proximal end of said longitudinal swab rod (2) on a pre-determined minimum length (l) and unfastened from each other towards a distal end of said longitudinal swab rod (2), each distal end (4.1) of said two sheets of fabric having a through hole (8) configured to allow attachment of each of the distal end (4.1) of said two sheets of fabric to said stopper means (6) during normal use of the swab applicator (1) and detachment of each of the distal end (4.1) of said two sheets of fabric from said stopper means (6) during analysis and evaluation of the specimens; each frangible wall (5.1, 5.2) further comprising at least an opening (7) according to claim 13, situated in the centre of the at least one rupture zone,

**such that** said frangible walls (5.1, 5.2) of said two sheets of fabric are strong enough to hold together said pouch and said two sheets of fabric during normal use of said swab applicator (1) and to rupture upon exertion of a tearing force (T) onto said distal end (4.1) of said cloth (4), in a direction opposite to the insertion direction (x) of the swab applicator (1), when the proximal end (4.2) of said cloth (4) is maintained in a fixed position relative to the distal end (4.1), thus separating the pouch from the two sheets of fabric.

18. Swab kit according to claim 17 wherein said pre-determined minimum width (w) of each of the frangible wall (5.1, 5.2) is within a range of about 9 mm to about 15 mm.

**Patentansprüche**

1. Abstrichset umfassend:

einen Abstrichapplikator (1) zur Entnahme einer Probe aus einer menschlichen oder tierischen Körperhöhle unter Verwendung eines wenig saugfähigen, flexiblen Probenahmetuches (4), umfassend:

- einen langgestreckten Abstrichstab (2) und

- einen länglichen Abstrichkopf (3), der an einem proximalen Ende (2.1) des Abstrichstabs (2) angeordnet ist

wobei der Abstrichapplikator (1) zumindest teilweise mit dem wenig saugfähigen, flexiblen Probenahmetuch (4) bedeckt ist und zum Einführen und Entfernen des Probenahmetuches (4) in die bzw. aus der Körperhöhle verwendet wird,

wobei das Probenahmetuch (4) ein distales Ende (4.1) und ein proximales Ende (4.2) aufweist, wobei das distale und das proximale Ende (4.1, 4.2) eine Einführungsrichtung (x) innerhalb einer menschlichen oder tierischen Körperhöhle mittels des Abstrichapplikators (1) definieren, wobei das proximale Ende (4.2) dasjenige Ende ist, das zuerst in die Körperhöhle eintritt, und das distale Ende (4.1) dasjenige Ende ist, das zuletzt in die Körperhöhle eintritt, wobei das Probenahmetuch (4) ferner mindestens einen Reißbereich umfasst, der zwischen einem oberen Bereich und einem unteren Bereich des Tuchs (4) angeordnet ist, wobei sich der obere Bereich vom proximalen Ende (4.2) in Richtung des Reißbereichs erstreckt und sich der untere Bereich vom Reißbereich in Richtung des distalen Endes (4.1) des Tuchs (4) in einer der Einführungsrichtung (x) in eine menschliche oder tierische Körperhöhle entgegengesetzten Richtung erstreckt, und wobei der obere Bereich des Tuchs (4) lösbar an dem Abstrichapplikator (1) befestigt ist und das Probenahmetuch (4) derart gestaltet ist, dass es an dem mindestens einen Reißbereich reißt, wenn eine Durchreißkraft (T) auf das distale Ende (4.1) des Tuchs (4) ausgeübt wird, während das proximale Ende (4.2) des Tuchs (4) in einer festen Position relativ zum distalen Ende (4.1) gehalten wird, wodurch der obere Bereich vom unteren Bereich getrennt wird

**dadurch gekennzeichnet, dass**

der langgestreckte Abstrichstab (2) und der längliche Abstrichkopf (3) an einem gebogenen Abschnitt (5) miteinander verbunden sind, wobei der gebogene Abschnitt (5) eine Neigung des länglichen Abstrichkopfes (3) ermöglicht, gemessen zwischen der mittleren Längsachse des länglichen Abstrichkopfes (3) und der mittleren Längsachse des Abstrichstabs (2), und dass ein Anschlagmittel (6) in der Nähe eines distalen Endes (2.2) am langgestreckten Abstrichstab (2) angeordnet ist, um das Probenahmetuch (4) während des Einführens und Entfernens des Applikators (1) aus der Körperhöhle an dem Abstrichapplikator (1) zu befestigen, wobei das Probenahmetuch (4) aus einem Gewebe mit einer Saugfähigkeit von 3,5 g/g oder weniger hergestellt ist, und der untere Bereich des Tuchs (4) Befestigungsmittel (8) umfasst, um das Probenahmetuch (4) während des Einführens und Entfernens des Applikators (1) in die bzw. aus der Körperhöhle an dem Abstrichapplikator (1) zu befestigen.

2. Abstrichset gemäß Anspruch 1, wobei die Neigung des länglichen Abstrichkopfes (3) einem Neigungsgrad von etwa 16% bis etwa 40% entspricht.

3. Abstrichset gemäß Anspruch 2, wobei der Anstieg (H) des Neigungsgrades in einem Bereich von etwa 5 mm bis etwa 10 mm liegt.

4. Abstrichset gemäß Anspruch 2 oder 3, wobei eine Länge (L) des Neigungsgrades in einem Bereich von etwa 25 mm bis etwa 30 mm, vorzugsweise etwa 30 mm, liegt.

5. Abstrichset gemäß einem der vorstehenden Ansprüche, wobei ein Neigungswinkel ($\alpha$), gemessen zwischen der mittleren Längsachse des länglichen Abstrichkopfes (3) und der mittleren Längsachse des Abstrichstabs (2), etwa 9° bis etwa 22° beträgt.

6. Abstrichset gemäß einem der vorstehenden Ansprüche, wobei der gebogene Abschnitt (5) vorzugsweise eine Bogenform mit einem Radius im Bereich von etwa 0,9 mm bis etwa 2,35 mm aufweist.

7. Abstrichset gemäß einem der vorstehenden Ansprüche, wobei der Abstrichapplikator (1) aus einem Kunststoffmaterial, vorzugsweise Polyester (Polyethylenterephthalat (PET)) oder ABS (Acrylnitril-Butadien-Styrol), hergestellt ist.

8. Abstrichset gemäß einem der vorstehenden Ansprüche, wobei das Probenahmetuch (4) aus einem Gewebe mit einer Dicke von 1 mm oder weniger, vorzugsweise 0,4 mm oder weniger, am meisten bevorzugt 0,2 mm oder weniger, hergestellt ist.

9. Abstrichset gemäß einem der vorstehenden Ansprüche, wobei das Probenahmetuch (4) aus einem Gewebe mit einem Flächengewicht von etwa 59,20 g/m2 oder weniger hergestellt ist.

10. Abstrichset gemäß einem der vorstehenden Ansprüche, wobei der obere Bereich des Probenahmetuches (4) aus einem Gewebe mit einem Gewicht von 0,1 g oder weniger hergestellt ist.

11. Abstrichset gemäß einem der vorstehenden Ansprüche, wobei der obere Bereich des Probenahmetuches (4) Folgendes umfasst:

zwei Gewebebahnen umfassend:

- einen proximalen Rand;
- einen distalen Rand,

wobei die Bahnen durch Befestigungsmittel (11) wie durchgehende Schweißnähte, Nähte und/oder Klebenähte miteinander verbunden sind, um eine Tasche mit einem offenen Ende, einer dem offenen Ende gegenüberliegenden und zum proximalen Rand hin gelegenen kurzen Seite und zwei entlang des proximalen Randes gelegenen lateralen Seiten zu bilden, wobei die kurze Seite der Tasche und die beiden lateralen Seiten in einem Abstand von mindestens 1 mm, vorzugsweise etwa 1 mm bis 8 mm, bevorzugt 1 mm bis 5 mm oder 1 mm bis 3 mm von den proximalen Rändern der Bahnen entfernt sind und zwei proximale Laschen (4b) bilden, die durch die Befestigungsmittel (11) an der kurzen Seite und an den lateralen Seiten aneinander befestigt sind und deren gegenüberliegendes Ende frei ist.

12. Abstrichset gemäß einem der vorstehenden Ansprüche, wobei der mindestens eine Reißbereich des Probenahmetuches (4) eine sich zu ihrer mittleren Querachse hin verjüngende Oberfläche aufweist, wodurch mindestens eine zerbrechliche Wand (5.1, 5.2) mit einer vorbestimmten Mindestbreite (w) proximal zur mittleren Querachse des mindestens einen Reißbereichs entsteht.

13. Abstrichset gemäß einem der vorstehenden Ansprüche, wobei das flexible Material des mindestens einen Reißbereichs des Probenahmetuches (4) mindestens eine Öffnung (7), vorzugsweise ein Durchgangsloch oder einen Schlitz oder eine Vielzahl von Löchern, aufweist, oder wobei der mindestens eine Reißbereich eine geringere Dicke im Vergleich zur Materialdicke der oberen und/oder unteren Bereiche aufweist, oder wobei der mindestens eine Reißbereich im Vergleich zum Material der oberen und/oder unteren Bereiche locker gewebt ist.

14. Abstrichset gemäß einem der vorstehenden Ansprüche derart gestaltet, dass die Durchreißkraft (T) eine seitliche oder lineare Kraft ist, die in einer zur Einführungsrichtung (x) des Abstrichapplikators entgegengesetzten Richtung oder in einer zur Einführungsrichtung (x) des Abstrichapplikators im Wesentlichen senkrechten Richtung und von der mittleren Längsachse des Abstrichapplikators weg ausgeübt wird, nach dem Entfernen des Abstrichapplikators (1) mit dem Probenahmetuch (4) aus der menschlichen oder tierischen Körperhöhle.

15. Abstrichset gemäß einem der vorstehenden Ansprüche, wobei der längliche Abstrichkopf (3) des Abstrichapplikators (1) vollständig vom proximalen Ende (4.2) des Probenahmetuches (4) während des Einführens und Entfernens des Abstrichapplikators (1) in die bzw. aus der Körperhöhle bedeckt ist und der gebogene Abschnitt (5) und der langgestreckte Abstrichstab (2) während des Einführens und Entfernens des Abstrichapplikators (1) in die bzw. aus der Körperhöhle teilweise von dem Probenahmetuch (4) bedeckt sind.

16. Abstrichset gemäß einem der vorstehenden Ansprüche, wobei der gebogene Abschnitt (5) proximal in Bezug auf den oberen Bereich des Probenahmetuches (4) angeordnet ist.

17. Abstrichset gemäß einem der vorstehenden Ansprüche, wobei das Probenahmetuch (4) Folgendes umfasst:

- zwei Gewebebahnen, jeweils umfassend:

- einen proximalen Rand, der sich an einem proximalen Ende des Abstrichkopfes (3) befindet,
- einen distalen Rand, der sich an einem distalen Ende (2.2) des langgestreckten Abstrichstabs (2) befindet,

wobei die Bahnen durch Befestigungsmittel (11) wie durchgehende Schweißnähte, Nähte und/oder Klebenähte miteinander verbunden sind, um eine Tasche mit einem offenen, proximal in Bezug auf den gebogenen Abschnitt (5) angeordneten Ende, einer dem offenen Ende gegenüberliegenden und zum proximalen Ende des Abstrichkopfes (3) hin angeordneten kurzen Seite und zwei entlang des proximalen Endes des Abstrichkopfes (3) angeordneten lateralen Seiten zu bilden, wobei die kurze Seite der Tasche und die beiden lateralen Seiten in einem Abstand von mindestens 1 mm, vorzugsweise von etwa 1 mm bis 8 mm, bevorzugt 1 mm bis 5 mm oder 1 mm bis 3 mm von den proximalen Rändern der Bahnen entfernt sind und zwei proximale Laschen (4b) bilden, die an der kurzen Seite und an den lateralen Seiten durch die Befestigungsmittel (11) aneinander befestigt sind und deren gegenüberliegendes Ende frei ist;
wobei sich jede Oberfläche der beiden Gewebebahnen in der Nähe mindestens eines Reißbereichs zur mittleren Querachse des Reißbereichs hin verjüngt, wodurch zwei zerbrechliche Wände (5.1, 5.2) mit einer vorbestimmten Mindestbreite (w) jeder der beiden Gewebebahnen proximal zur mittleren Querachse des Reißbereichs entstehen;

wobei die beiden Gewebebahnen durch die Befestigungsmittel (11) entlang eines proximalen Endes des langgestreckten Abstrichstabs (2) auf einer vorbestimmten Mindestlänge (l) weiter aneinander befestigt sind und in Richtung eines distalen Endes des langgestreckten Abstrichstabs (2) voneinander gelöst sind, wobei jedes distale Ende (4.1) der beiden Gewebebahnen ein Durchgangsloch (8) aufweist, das derart gestaltet ist, dass es die Befestigung jedes distalen Endes (4.1) der beiden Gewebebahnen an dem Anschlagmittel (6) während der normalen Verwendung des Abstrichapplikators (1) und die Trennung jedes distalen Endes (4.1) der beiden Gewebebahnen von dem Anschlagmittel (6) während der Analyse und Auswertung der Proben ermöglicht;
wobei jede zerbrechliche Wand (5.1, 5.2) ferner mindestens eine Öffnung (7) gemäß Anspruch 13 umfasst, die sich in der Mitte des mindestens einen Reißbereichs befindet,

**so dass** die zerbrechlichen Wände (5.1, 5.2) der beiden Gewebebahnen stark genug sind, um die Tasche und die beiden Gewebebahnen während der normalen Verwendung des Abstrichapplikators (1) zusammenzuhalten und bei Ausübung einer Durchreißkraft (T) auf das distale Ende (4.1) des Tuchs (4) zu brechen, in einer der Einführungsrichtung (x) des Abstrichapplikators (1) entgegengesetzten Richtung, wenn das proximale Ende (4.2) des Tuchs (4) in einer festen Position relativ zum distalen Ende (4.1) gehalten wird, wodurch die Tasche von den beiden Gewebebahnen getrennt wird.

18. Abstrichset gemäß Anspruch 17, wobei die vorbestimmte Mindestbreite (w) jeder der zerbrechlichen Wände (5.1, 5.2) in einem Bereich von etwa 9 mm bis etwa 15 mm liegt.

**Revendications**

1. Kit d'écouvillon comprenant:

un applicateur d'écouvillon (1) destiné à prélever un échantillon dans une cavité corporelle humaine ou animale à l'aide d'un tissu d'échantillonnage souple à faible pouvoir absorbant (4), comprenant:

- une tige d'écouvillon longitudinale (2) et
- une tête d'écouvillon allongée (3) disposée à une extrémité proximale (2.1) de ladite tige d'écouvillon (2)

dans lequel ledit applicateur d'écouvillon (1) est au moins partiellement recouvert dudit tissu d'échantillonnage souple à faible pouvoir absorbant (4) et utilisé pour insérer et retirer le tissu d'échantillonnage (4) dans et hors de la cavité corporelle,
le tissu d'échantillonnage (4) ayant une extrémité distale (4.1) et une extrémité proximale (4.2), lesdites extrémités distale et proximale (4.1, 4.2) définissant une direction d'insertion (x) à l'intérieur d'une cavité corporelle humaine ou animale au moyen de l'applicateur d'écouvillon (1), dans lequel ladite extrémité proximale (4.2) est l'extrémité qui pénètre en premier dans la cavité corporelle et ladite extrémité distale (4.1) est l'extrémité qui pénètre en dernier dans la cavité corporelle, ledit tissu d'échantillonnage (4) comprenant en outre au moins une zone de rupture positionnée entre une zone supérieure et une zone inférieure dudit tissu (4), dans lequel ladite zone supérieure s'étend de l'extrémité proximale (4.2) vers la zone de rupture et ladite zone inférieure s'étend de la zone de rupture vers l'extrémité distale (4.1) dudit tissu (4) dans une direction opposée à l'insertion (x) à l'intérieur d'une cavité corporelle humaine ou animale, et ladite zone supérieure du tissu (4) est fixée de manière amovible audit applicateur d'écouvillon (1) et le tissu d'échantillonnage (4) est configuré pour se rompre au niveau de ladite au moins une zone de rupture lorsqu'une force de déchirure (T) est exercée sur ladite extrémité distale (4.1) dudit tissu (4), lorsque l'extrémité proximale (4.2) dudit tissu (4) est maintenue dans une position fixe par rapport à l'extrémité distale (4.1), séparant ainsi la zone supérieure de la zone inférieure
**caractérisé en ce que**
la tige d'écouvillon longitudinale (2) et la tête d'écouvillon allongée (3) sont reliées l'une à l'autre au niveau d'une section courbée (5), dans laquelle la section courbée (5) permet une inclinaison de la tête d'écouvillon allongée (3) mesurée entre l'axe longitudinal central de ladite tête d'écouvillon allongée (3) et l'axe longitudinal central de ladite tige d'écouvillon (2), et **en ce qu'**un moyen d'arrêt (6) est disposé à proximité d'une extrémité distale (2.2) sur la tige d'écouvillon longitudinale (2) pour fixer le tissu d'échantillonnage (4) audit applicateur d'écouvillon (1) pendant l'insertion et le retrait de l'applicateur (1) de la cavité corporelle, dans lequel le tissu d'échantillonnage (4) est constitué d'un tissu ayant une capacité d'absorption de 3,5 g/g ou moins, et ladite zone inférieure du tissu (4) comprend des moyens de fixation (8) pour fixer le tissu d'échantillonnage (4) audit applicateur d'écouvillon (1) pendant l'insertion et le retrait de l'applicateur (1) dans et hors de la cavité corporelle.

**2.** Kit de d'écouvillon selon la revendication 1, dans lequel ladite inclinaison de la tête d'écouvillon allongée (3) correspond à un pourcentage de pente d'environ 16% à environ 40%.

**3.** Kit d'écouvillon selon la revendication 2, dans lequel une valeur de la hauteur (H) dudit pourcentage de pente est comprise dans une plage d'environ 5 mm à environ 10 mm.

**4.** Kit d'écouvillon selon la revendication 2 ou 3, dans lequel une valeur de la longueur (L) dudit pourcentage de pente est comprise dans une plage d'environ 25 mm à environ 30 mm, plus préférablement d'environ 30 mm.

**5.** Kit d'écouvillon selon l'une quelconque des revendications précédentes, dans lequel l'angle d'inclinaison ($\alpha$) mesuré entre l'axe longitudinal central de ladite tête d'écouvillon allongée (3) et l'axe longitudinal central de ladite tige d'écouvillon (2) est compris entre environ 9° et environ 22°.

**6.** Kit d'écouvillon selon l'une quelconque des revendications précédentes, dans lequel ladite section courbée (5) a de préférence une forme d'arc avec un rayon compris dans une plage d'environ 0,9 mm à environ 2,35 mm.

**7.** Kit d'écouvillon selon l'une quelconque des revendications précédentes, dans lequel l'applicateur d'écouvillon (1) est fabriqué en matière plastique, de préférence en polyester (polyéthylène téréphtalate (PET)) ou en ABS (acrylonitrile butadiène styrène).

**8.** Kit d'écouvillon selon l'une quelconque des revendications précédentes, dans lequel ledit tissu d'échantillonnage (4) est constitué d'un tissu ayant une épaisseur de 1 mm ou moins, de préférence de 0,4 mm ou moins, et le plus préférablement de 0,2 mm ou moins.

**9.** Kit d'écouvillon selon l'une quelconque des revendications précédentes, dans lequel ledit tissu d'échantillonnage (4) est constitué d'un tissu ayant un grammage d'environ 59,20 g/m2 ou moins.

**10.** Kit d'écouvillon selon l'une quelconque des revendications précédentes, dans lequel la zone supérieure dudit tissu d'échantillonnage (4) est constituée d'un tissu ayant un poids inférieur ou égal à 0,1 g.

**11.** Kit d'écouvillon selon l'une quelconque des revendications précédentes, dans lequel la zone supérieure dudit tissu d'échantillonnage (4) comprend:

deux feuilles de tissu, chacune ayant:

- un bord proximal;
- un bord distal,

dans lequel lesdites feuilles sont fixées l'une à l'autre par des moyens de fixation (11) tels que des lignes de soudure continues, des lignes de couture et/ou des lignes de colle pour former une poche ayant une extrémité ouverte, un côté court opposé à l'extrémité ouverte et situé vers le bord proximal, et deux côtés latéraux situés le long de ledit bord proximal, dans laquelle ledit côté court de la poche et lesdits deux côtés latéraux sont situés à une distance d'au moins 1 mm, de préférence d'environ 1 mm à 8 mm, plus préférablement entre 1 mm et 5 mm, ou entre 1 mm et 3 mm des bords proximaux desdites feuilles, formant deux rabats proximaux (4b) fixés l'un à l'autre par les moyens de fixation (11) au niveau du côté court et des côtés latéraux et ayant l'extrémité opposée libre.

**12.** Kit d'écouvillon selon l'une quelconque des revendications précédentes, dans lequel la au moins une zone de rupture dudit tissu d'échantillonnage (4) présente une surface qui se rétrécit vers son axe transversal central, ce qui donne au moins une paroi fragile (5.1, 5.2) ayant une largeur minimale prédéterminée (w) à proximité de l'axe transversal central de la au moins une zone de rupture.

**13.** Kit d'écouvillon selon l'une quelconque des revendications précédentes, dans lequel le matériau souple de la au moins une zone de rupture dudit tissu d'échantillonnage (4) comprend au moins une ouverture (7), de préférence un trou traversant ou une fente ou une pluralité de perforations, ou dans lequel la au moins une zone de rupture a une épaisseur inférieure à l'épaisseur du matériau des zones supérieure et/ou inférieure, ou dans lequel la au moins une zone de rupture est tissée de manière lâche par rapport au matériau des zones supérieure et/ou inférieure.

**14.** Kit d'écouvillon selon l'une quelconque des revendications précédentes, configuré de telle sorte que ladite force de déchirure (T) est une force latérale ou linéaire exercée dans une direction opposée à la direction d'insertion (x) de l'applicateur d'écouvillon ou dans une direction sensiblement perpendiculaire à la direction d'insertion (x) de l'applicateur d'écouvillon et éloignée de l'axe longitudinal central dudit applicateur d'écouvillon, après retrait de l'applicateur d'écouvillon (1) comprenant le tissu d'échantillonnage (4) de la cavité corporelle humaine ou animale.

**15.** Kit d'écouvillon selon l'une quelconque des revendications précédentes, dans lequel ladite tête d'écouvillon allongée (3) de l'applicateur d'écouvillon (1) est entièrement recouverte par ladite extrémité proximale (4.2) du tissu d'échantillonnage (4) pendant l'insertion et le retrait de l'applicateur d'écouvillon (1) dans et hors de la cavité corporelle, et ladite section courbée (5) et ladite tige d'écouvillon longitudinale (2) sont partiellement recouvertes par ledit tissu d'échantillonnage (4) pendant l'insertion et le retrait de l'applicateur d'écouvillon (1) dans et hors de la cavité corporelle.

16. Kit d'écouvillon selon l'une quelconque des revendications précédentes, dans lequel la section courbée (5) est positionnée de manière proximale par rapport à ladite zone supérieure du tissu d'échantillonnage (4).

17. Kit d'écouvillon selon l'une quelconque des revendications précédentes, dans lequel ledit tissu d'échantillonnage (4) comprend:

   - deux feuilles de tissu, chacune ayant:

      - un bord proximal situé à une extrémité proximale de la tête d'écouvillon (3),
      - un bord distal situé à une extrémité distale (2.2) de la tige d'écouvillon longitudinale (2),

   dans lequel lesdites feuilles sont fixées l'une à l'autre par des moyens de fixation (11) tels que des lignes de soudure continues, des lignes de couture et/ou des lignes de colle pour former une poche ayant une extrémité ouverte située de manière proximale par rapport à la section courbée (5), un côté court opposé à l'extrémité ouverte et situé vers l'extrémité proximale de la tête d'écouvillon (3), et deux côtés latéraux situés le long de ladite extrémité proximale de la tête d'écouvillon (3), dans lequel ledit côté court de la poche et lesdits deux côtés latéraux sont situés à une distance d'au moins 1 mm, de préférence d'environ 1 mm à 8 mm, plus préférablement entre 1 mm et 5 mm, ou entre 1 mm et 3 mm des bords proximaux desdites feuilles, formant deux rabats proximaux (4b) fixés l'un à l'autre par les moyens de fixation (11) au niveau du côté court et des côtés latéraux et ayant l'extrémité opposée libre;
   chaque surface des deux feuilles de tissu se rétrécit à proximité d'au moins une zone de rupture vers l'axe transversal central de la zone de rupture, ce qui donne deux parois fragiles (5.1, 5.2) ayant une largeur minimale prédéterminée (w) de chacune des deux feuilles de tissu à proximité de l'axe transversal central de la zone de rupture;
   lesdites deux feuilles de tissu sont en outre fixées l'une à l'autre par les moyens de fixation (11) le long d'une extrémité proximale de ladite tige d'écouvillon longitudinale (2) sur une longueur minimale prédéterminée (l) et détachées l'une de l'autre vers une extrémité distale de ladite tige d'écouvillon longitudinale (2), chaque extrémité distale (4.1) desdites deux feuilles de tissu comportant un trou traversant (8) configuré pour permettre la fixation de chacune des extrémités distales (4.1) desdites deux feuilles de tissu audit moyen d'arrêt (6) pendant l'utilisation normale de l'applicateur d'écouvillon (1) et le détachement de chacune des extrémités distales (4.1) desdites deux feuilles de tissu dudit moyen d'arrêt (6) pendant l'analyse et l'évaluation des échantillons;
chaque paroi fragile (5.1, 5.2) comprenant en outre au moins une ouverture (7) selon la revendication 13, située au centre de la au moins une zone de rupture,

**de telle sorte** que lesdites parois fragiles (5.1, 5.2) desdites deux feuilles de tissu soient suffisamment solides pour maintenir ensemble ladite poche et lesdites deux feuilles de tissu pendant l'utilisation normale dudit applicateur d'écouvillon (1) et pour se rompre lors de l'application d'une force de déchirure (T) sur ladite extrémité distale (4.1) dudit tissu (4), dans une direction opposée à la direction d'insertion (x) de l'applicateur d'écouvillon (1), lorsque l'extrémité proximale (4.2) dudit tissu (4) est maintenue dans une position fixe par rapport à l'extrémité distale (4.1), séparant ainsi la poche des deux feuilles de tissu.

18. Kit d'écouvillon selon la revendication 17, dans lequel ladite largeur minimale prédéterminée (w) de chacune des parois fragiles (5.1, 5.2) est comprise dans une plage d'environ 9 mm à environ 15 mm.

FIG. 1

FIG. 2

Fig. 3a

Fig. 3b

Fig. 4b

Fig. 4a

Fig. 5a

Fig. 5b

Fig. 5c

Fig. 6a

Fig. 6b

Fig. 6c

Fig. 6d

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 204106055 U
- CN 205215271 U
- CN 206818436 U
- WO 1999021521 A1
- US 20200390425 A1
- US 20180161019 A1
- US 20180353737 A1
- DE 202009016553 U1
- US 5121752 A
- US 2010286552 A1